# EUROPÄISCHE PATENTANMELDUNG

(11) **EP 2 487 490 A1**
(43) Veröffentlichungstag der Anmeldung: **15.08.2012**
(21) Anmeldenummer: 11154128.0
(22) Anmeldetag: 11.02.2011
(51) Int. Cl.: G01N 33/544, G01N 33/552

(54) **Heterogener Bindungsassay mit verbesserter optischer Auswertbarkeit oder poröse Festphase für Bindungsassays mit verbesserter optischer Auswertbarkeit**

(71) Anmelder: FZMB GmbH Forschungszentrum für Medizintechnik und Biotechnologie, 99947 Bad Langensalza (DE)
(72) Erfinder: Miethe, Peter, 06632 Schleberoda (DE); Henze, Stephan, 99096 Erfurt (DE)
(74) Vertreter: von Kreisler Selting Werner

(57) **Zusammenfassung**

Poröser Träger für heterogene Bindungsassays, wobei der Träger Poren aufweist, die ein Porenvolumen besitzen und die Poren in einer festen Phase angeordnet sind, mit der Maßgabe, dass die feste Phase einen Brechungsindex aufweist und das Porenvolumen einen Brechungsindex aufweist, dadurch gekennzeichnet, dass eine Differenz des Brechungsindex der festen Phase und des Brechungsindex des Porenvolumens < 0,05, insbesondere <0,02 ist.

## Beschreibung

Die Erfindung betrifft einen porösen Träger für heterogene Bindungsassays.

In den letzten drei Dekaden sind eine Reihe von einfachen Schnelltestsystemen für den Nachweis von Analyten in Körperflüssigkeiten, Prozessflüssigkeiten, Umweltproben und Lebensmittelproben auf Basis von heterogenen Bindungsassays entwickelt worden. Von herausragender Bedeutung sind dabei Assays bei denen spezifische Bindungsmoleküle (Rezeptoren) auf porösen saugfähigen oder nicht saugfähigen Membranen oder Formstücken immobilisiert und Analyten im Durchfluss in einer definierten, zumeist banden- oder punktförmigen Reaktionszone spezifisch an diese gebunden werden. Typische Produkte dieser Art sind "lateral flow" Teststreifen wie in US-B-6,485,982, Durchflussmembranassays, wie in EP-B-207 152 oder WO-A1-87/07384 und auf Mikrosäulen basierende Testformate, wie in EP-A-0634015 beschrieben. Die Detektion des Bindungsereignisses erfolgt durch vorherige oder nachfolgende Zugabe einer mit dem Analyt reagierenden Markierung. Von herausragender praktischer Bedeutung sind dabei visuell oder mit Hilfe einer einfachen optischen Auslesevorrichtung erfassbare Markierungen wie Anti-Analyt-Goldpartikel-Konjugate, Anti-Analyt-Farbpartikel-Konjugate, Anti-Analyt-Fluoreszenzfarbstoff-Konjugate, Anti-Analyt-Fluoreszenzfarbstoffpartikel-Konjugate, Anti-Analyt-Enzym- Konjugate in Kombination mit präzipitierenden Substraten. Typische Anti-Analyten sind Antikörper, immunologisch aktive Antigene, Streptavidin, Protein A und G, Lectine, Oligonekleotide und Oligosaccaride. Diese Grundtechniken werden auch in einem sogenannten kompetitiven Testformat, genutzt wobei hier auch Konjugate aus Analyt und einer Markierung eingesetzt werden.

Derzeit werden zur Herstellung dieser Testvorrichtungen ausschließlich weiße, stark lichtstreuende Membranen auf Basis von Nitrozellulose, Polyethylen, Nylon, Cellulose, Celluloseacetat, Papier eingesetzt. Die Bindung der Markierungselemente an den Analyt wird auf diesen Materialen durch gefärbte oder fluoreszierende Banden bzw. Punkte sichtbar. Da mit dem bloßen Auge keine verlässliche Quantifizierung möglich ist, wurden zur Auswertung eine Reihe von optischen Lesegeräten entwickelt, die entweder im Reflexionsmodus oder im Transmissionsmodus arbeiten. Damit kann bei Einsatz einer Farbstoffmarkierung die durch die Markierung verursachte Schwächung des eingestrahlten Lichtes erfasst und zur Quantifizierung des Assays eingesetzt werden. Mit diesen Testsystemen können Nachweisgrenzen erreicht werden, die für praktisch relevante Proteine wie Troponin I, CRP, D-Dimer im Bereich von einigen ng/ml liegen. Diese relativ hohe Nachweisgrenze ist bei der Reflektrometrie messtechnisch durch die geringe Eindringtiefe des Lichtes begründet. Typische Werte liegen bei 100 µm. Im Transmissionsmodus, bei dem die Auswertung auf einem modifizierten Lambert-Beerschen-Gesetz beruht, begrenzen der natürliche Extinktionskoeffizient der Lichtabsorption der Marker und die Schichtdicke der Membranen die Erfassbarkeit der Farbmarkierung durch optische Verfahren. Außerdem müssen weiße, lichtstreuende Membranen und Träger individuell referenziert werden, da bereits geringe Inhomogenitäten im Material große Schwankungen in der optischen Dichte erzeugen können, wodurch solche Messverfahren gerätetechnisch aufwändig sind. Auch mit modernen bildgebenden Verfahren können keine wesentlich sensitiveren Verfahren realisiert werden, da nur ausreichend große Markierungen erfasst werden können, die sich an der Oberfläche der Membran befinden.

Fluorimetrische Markierungen, insbesondere in Form von fluoreszenten Partikeln lassen prinzipiell wesentlich niedrigere Nachweisgrenzen zu. Um diese Farbstoffe quantitativ erfassen zu können, ist es notwendig eine Messvorrichtung einzusetzen, bei der das emittierte Licht quantitativ ohne Störung durch das Anregungslicht erfasst wird. Dies wird in Fluorimetern technisch dadurch erreicht, dass geometrische Anordnungen gewählt werden, wie beispielsweise einem Winkel von 90° zwischen Anregungsstrahl und Emissionsmessrichtung, die eine geometrische Trennung der beiden Lichtanteile zulassen. Bei stark streuenden Materialien, wie den oben beschriebenen, ist eine derartige Anordnung nicht vorteilhaft nutzbar, da Anregungs- und Fluoreszenzlicht gleichartig in alle Raumrichtungen remittiert werden. Zur Trennung von Anregungs- und Emissionslicht gibt es hier, insbesondere bei gebräuchlichen Fluoreszenzmarkierungen wie Fluorescein, Phycocyanin, Phycoerythrin nur die Möglichkeiten optische Filter mit steiler Kantencharakteristik und hoher Extinktion einzusetzen. Diese Filter sind teuer, fragil, stellen hohe Anforderungen an die Präzision des optischen Aufbaus und machen das Gesamtverfahren wirtschaftlich unattraktiv.

Eine Aufgabe der vorliegenden Erfindung ist es, einen Träger zur Verfügung zu stellen, der die oben genannten Nachteile vermeidet. Eine weitere Aufgabe besteht darin, dass der Träger einen quantitativen und qualitativen heterogenen Bindungsassay zum schnellen Nachweis von gelösten Analyten, wie Proteinen, Hormonen, Arzneimitteln, Polypeptiden, Glycosiden, Nukleinsäuren, Viren, Zellen mit verbesserter optischer Auswertung ermöglicht.

Diese Aufgaben werden mit dem erfindungsgemäßen Träger gelöst. Der erfindungsgemäße Träger für heterogene Bindungsassays ist ein poröser Träger, der immobilisierte Bindungsmoleküle zum spezifischen Binden von Analyten oder Konjugaten sowie Poren aufweist, die ein Porenvolumen besitzen und die Poren in einer festen Phase angeordnet sind, mit der Maßgabe, dass die feste Phase einen Brechungsindex aufweist und das Porenvolumen einen Brechungsindex aufweist, dadurch gekennzeichnet, dass eine Differenz des Brechungsindex der festen Phase und des Brechungsindex des Porenvolumens kleiner als 0,05 insbesondere kleiner als 0,02 ist.

Bindungsmoleküle im Sinne der vorliegenden Erfindung sind alle aus konventionellen Bindungsassays sowie der Affinitätschromatographie bekannten biologischen und synthetischen Liganden, die mit dem nachzuweisenden Analyt oder einem der Probe hinzugefügtem, für den Analyt spezifischen Bindungsmolekül, bevorzugt vor anderen Bestandteilen der Probe eine Bindung eingehen. Dies sind insbesondere natürliche oder rekombinate Antikörper, Antikörperfragmente und - mimetika, immunologisch aktive Antigene, Oligonukleotide, Poly- und Oligosccaride, Poly- und Oligosaccarid bindende Moleküle wie Lektine, Liganden nachzuweisender Rezeptormoleküle, Cofaktoren nachzuweisender Enzyme, Bakteriophagen oder -proteine, IgG bindende Proteine wie Protein G, A oder L sowie Bindungspartner spezifischer Affinitätstags, wie Step-tag, His-Tag und ähnliche. Darüber hinaus sind auch Liganden mit hydrophoben, anionischen, kationischen oder zwitterionischen Eigenschaften oder durch molekulares Imprinting erzeugte Oberflächenstrukturen einsetzbar, wenn sie durch geeignete Wahl der Bindungsbedingungen ein zumindest teilselektives Binden für den nachzuweisenden Analyt zulassen. Weitere Beispiele für Bindungsmoleküle finden sich z.B. in Taylor & Francis, ed. By David S. Hage, Affinity Chromatography (2006), Vol. 92.
Figur 1 zeigt eine Filteranordnung und Auswerteoptik.
Figur 2 zeigt eine Kalibrationskurve (Troponin I) des Durchfluss Membran-Assays entsprechend Beispiel 1.
Figur 3 zeigt eine Filteranordnung und Auswerteoptik.
Figur 4 zeigt eine typische Kalibrationskurve des Troponin I Lateral-Flow-Test entsprechend Beispiel 2.
Figur 5 zeigt eine Durchflussanordnung für Miniatursäulen.
Figur 6 zeigt eine typische Kalibrationskurve des Säulen Durchfluss-Assays entsprechend Beispiel 3.

Das Angleichen der Brechungsindices von Poren und Festphase kann dabei erfindungsgemäß dadurch erreicht werden, dass die Pore mit einer Flüssigkeit gefüllt ist, deren Brechungsindex dem der Festphase entspricht. Dies kann durch eine geeignete Auswahl der im Assays verwendeten Reagenzien oder durch Austausch des Poreninhaltes nach Abschluss des Bindungsassays erfolgen.

In einem ersten Fall kann dem Assayreagenz zum Beispiel eine seine Brechzahl erhöhende, mit Wasser mischbare oder in Wasser lösliche Substanz in der Konzentration zugesetzt werden, um den Brechungsindex des Gemisches dem der Festphase anzugleichen. Besonders geeignete Substanzen sind wassermischbare Flüssigkeiten hoher Dichte, wie z.B. Glycerin, kurzkettige Polyethylenglycole, Thiodiglycol oder wasserlösliche Feststoffe wie Salze (Natriumiodid, Cäsiumchlorid) oder Polysaccharide (Saccharose, Trehalose). Dabei sollte die Konzentration der zugesetzten Substanzen so niedrig bleiben, dass der Assay, z.B. die Bildung von des Analyt-Bindungsmolekül-Komplexes nicht behindert wird. Wenn die Festphase überwiegend aus Substanzen mit einem Brechungsindex im Berei ch von +/- 0,05 insbesondere +/- 0,02 des Brechungsindexes der verwendeten wässrigen Pufferlösung besteht kann diese auch ohne Zusätze eingesetzt werden.

In einem anderen Fall kann nach Beendigung des Bindungsassays das zuletzt zugefügte Assayreagenz durch eine Flüssigkeit verdrängt werden, deren Brechungsindex dem der Festphase entspricht. Hierfür sind zunächst die gleichen wässrigen Gemische und Lösungen wie im vorigen Absatz beschrieben geeignet. Die Konzentration, in der die Substanz zugesetzt werden kann, ist jedoch weniger eingeschränkt. Die Bildung der Analyt-Bindungsmolekül-Komplexe darf sogar eventuell verhindert werden. Die Bindung bereits gebildeter Komplexe oder die durch diese erzeugten Anfärbungen sollte jedoch bestehen bleiben. So kann beispielsweise bei Verwendung von fluoreszenzmarkierten Antikörpern auf konventionellen, gesinterten Polyethylenträgern Thiodiglycol in Reinform verwendet werden. Sofern eine hinreichende Benetzbarkeit der Festphase gegeben ist, können auch nicht mit Wasser mischbare Substanzen, wie z.B. Immersionsöle, verwendet werden.

Der erfindungsgemäße Träger gestattet insbesondere auch die Durchführung heterogener Bindungsassays mit fluorimetrischer Auswertung ohne Verwendung von wellenlängenselektiven Elementen, wenn die Fluoreszenzlebensdauer der Markierung ausgewertet wird. Hierbei erfolgt die Selektion des Fluoreszenzlichtes anhand der charakteristischen Fluoreszenzlebensdauer der eingesetzten Markierung, insbesondere mittels der Frequenzmodulationsmethode oder der Timegate-Integratiosmethode. Die günstigen optischen Eigenschaften des Trägers stellen hierbei die erfoderliche dynamische Reserve der optischen Detektion sicher. Derartige Messungen sind bisher nur in homogenen Phasen oder unter Verwendung von optischen Filtern zur Trennung von Anregungs und Fluoreszenzlicht etabliert.

In einer Ausführungsform, bei der die Festphase einen Brechungsindex nahe dem wässriger Lösungen aufweist, kann der erfindungsgemäße Träger aus einem porösen Polymermaterial, insbesondere aus fluorierten oder perfluorierten Polymeren, wie perfluoriertes Ethlen-Propylen-Copolymer (FEP), Polytetrafluoroethylen (PTFE), Hexafluoropropylenvinylidenefluorid (THV), Perfluoroalkoxyalkanen (PFA), sulfoniertem Polytetrafluorethylen (NAFION), oder auch nanoporösen Siliziumdioxid bestehen. In einer weiteren Bauform, bei der eine Brechzahländerung durch Austausch der der flüssigen Phase erfolgt, kann der Träger beispielsweise aus Polyethylen, Cellulose, Nitrozellulose, Polyamid, Polymethacrylat, mineralische Gläsern bestehen.

Der erfindungsgemäße Träger, der ein Porenvolumen besitzt, kann auf unterschiedliche Weise hergestellt werden. Geeignet sind insbesondere Sinterverfahren, bei denen ein partikuläres Polymermaterial unter Erwärmung in eine Form gepresst wird. Alternativ kann auch ein fibrilläres Polymermaterial durch eine erwärmte Düse oder einen Spalt gezogen werden. Darüber hinaus sind auch Fällverfahren wie sie beispielsweise zur Herstellung von Zellulose- oder Nitrozellulosemembrane und solche, die zur Herstellung von Vliesen genutzt werden oder Webtechnologien einsetzbar. Außerdem sind porenerzeugende Verfahren geeignet, wie insbesondere die Expansion des Ausgangsmaterials durch flüchtige Treibmittel (Aufschäumen) oder durch lösliche Opfermaterialien, die vor der Verwendung des Trägers aus den Poren herausgelöst werden. Neben diesen Material umformenden Methoden sind auch solche Methoden geeignet, bei denen eine Porenbildung während des Abscheidens der Festphase aus einem Lösungsmittel stattfindet, wie zum Beispiel Hydrogel- Cryogel- oder Aerogel- Verfahren.

Die Größe der Poren kann vom Fachmann in Abhängigkeit vom Assay gewählt werden. Typischerweise liegen bei den erfindungsgemäß verwendbaren Trägern die Porengrößen im Bereich von 0,5 bis 5 µm bei lateral flow und 1 bis 50 µm bei Durchflussassays.

Die Verwendung solcher Art aufeinander abgestimmter Träger ermöglicht die Bereitstellung heterogener Bindungsassays, bei denen eine Auswertung durch Erfassen von Färbungen oder Fluoreszenzen in der gesamten Reaktionszone des porösen Trägers oder durch die Erfassung von gefärbten oder fluoreszierenden Einzelpartikeln erfolgen kann. Mit dem erfindungsgemäßen Träger können auch heterogene Bindungsassays eingesetzt werden, bei denen fluoreszierende Marker mit einfachen Messvorrichtungen ausgewertet werden können. Mit diesen Verfahren ist es möglich, eine Reihe von heterogenen Testverfahren, insbesondere die oben zitierten lateral flow Teststreifen, Durchflussassays und Säulenassays aufzubauen, mit Nachweisgrenzen, die bis zu 3 log Stufen niedriger sind als die von Verfahren auf Basis von konventionellen Materialien.

Als Bindungsmoleküle kommen alle bei heterogenen Bindungsassays in bekanntem Umfang einsetzbaren Moleküle in Frage. Insbesondere bewährt haben sich hierbei Antikörper, die als Bindungsmoleküle praktisch alle möglichen Analyten durch Affinitätsbindung zu Binden vermögen. Grundsätzlich kommen aber auch andere Bindungsmoleküle in Betracht, wie immunologisch aktive Antigene, Protein A/G, Antikörperfragmente, Lektine, Bakteriophagen bzw. Bakteriophagenproteine, Polysaccharide, Lipopolysaccaride und Oligonukleotide.

Eine Immobilisierung dieser Bindungsmoleküle, die aufgrund ihrer chemischen Beschaffenheit mit funktionalen Gruppen der ggf. funktionalisierten porösen Träger aus Polymeren, covalente Bindungen eingehen können, kann durch chemische Reaktion entsprechender Gruppen in den Bindungsmolekülen mit den komplementären Gruppen am porösen Trägermaterial erfolgen.

Das für den Träger vorgesehene poröse Material kann, sofern an dessen Oberfläche keine funktionalen Gruppen zur chemischen Kopplung von Bindungsmolekülen vorhanden sind, durch chemische Reaktionen mit geeigneten Mitteln funktionalisiert werden, um für den durchzuführenden heterogenen Bindungsassay erforderliche Bindungsmoleküle an dem porösen Träger zu immobilisieren.

So kann beispielsweise das poröse Trägermaterial einer Plasmabehandlung unterzogen werden. Hierbei wird es bei einem Druck von ca. 0,5 mBar Wasserdampf einem Hochfrequenzplasma ausgesetzt, wodurch temporäre -OH Funktionalitäten an der Oberfläche entstehen, über die nachfolgend eine Oberflächenmodifizierung erfolgen kann.

Alternativ können als Membran ausgebildete poröse Träger auf PTFE - Basis auch entsprechend DE-A-10 2009 003374, "Permanente hydrophile poröse Überzüge auf einem Substrat und poröse Membranen daraus", Absatz 0061 und Tabelle 1 mit einer hydrophylisierenden Beschichtung aus Polyvinylalkohol-Methacrylat (PV-MMA(2,5)) versehen werden.

Eine so durch OH-Gruppen hydrophilisierte PTFE Membran kann beispielsweise weiter in organischen Lösungsmittel mit 3-Aminopropyl-triethoxysilan umgesetzt und so mit Aminogruppen versehen werden. Diese können genutzt werden um daran mit der in Handbook of Affinity Chromatography (2006), Vol. 92, Taylor & Francis, edited by David S Hage S.54-55 beschrieben Methode, die auf homobifunktionellen N-Hydroxysuccinimidestern basiert, Rezeptoren zu koppeln. Der Einsatz von Bernsteinsäure als bifunktioneller Spacer ist dabei besonders vorteilhaft.

Mit dieser Methode lassen sich beispielsweise Antikörper an Membranen aus hydrophilisiertem PTFE (OMNIPORE JC von Millipore) koppeln. Typische Modifizierungsgrade liegen dabei im Bereich von 1-100 µg Antikörper, insbesondere bei 5-10 µg Antikörper pro 100 µl Membranvolumen. Bei einer Membrandicke von 50 µm entspricht der obere Wert dieses optimale Bereichs etwa 4µg Antikörper pro 1cm² Filterfläche mit etwa bis 3x 10¹³ spezifischen Bindungsstellen (binding site). Da die Kopplungsausbeuten bei dem Verfahren typischerweise im Bereich von 1-10 % liegen kann man von etwa 0,3-3x10¹² Bindungsstellen pro cm² ausgehen. Bei Verwendung von Materialien mit größerer innerer Oberfläche können aber auch wesentlich höhere spezifische Bindungskapazitäten erreicht werden.

Alternativ ist es auch möglich, Rezeptoren durch Adsorption auf Träger aufzubringen. Eine besonders einfache Technik, die bei Polyethylensintermaterial anwendbar ist, besteht darin, das poröse Polymermaterial entsprechend (Meyer M.F, et al, Biosensors and Bioelectronics 2007,22(6)p.973) mehrfach mit Ethanol zu behandeln und anschließend mit dem Proteinrezeptor in einem leicht alkalischen Puffer zu inkubieren.

Die Erfindung wird an Hand der folgenden Beispiele näher erläutert.

### Beispiel 1 Fluorimetrischer Nachweis von Troponin I im Durchflussverfahren

### 1. Funktionalisierung einer PTFE-Membran und Antikörperimmobilisierung

Im ersten Schritt wurde eine mit OH-Gruppen hydrophilisierte PTFE Membran (OMNIPORE JC von Millipore) durch Behandlung mit 3-Aminopropyl-triethoxysilan (APTS) aminiert. Dazu wurden 50 µl 3-Aminopropyl-triethoxysilan (Sigma-Aldrich Chemie GmbH) in 4950 µl Aceton gelöst und in einer gläsernen 90mm Petrischale auf die trockene Membran (d= 5cm) gegeben. Die Petrischale wurde in einen gläsernen Exsikkator überführt und dieser zügig (<10min) auf <300mbar evakuiert. Im verschlossenen Exsikkator wurde auf einem Kreisschüttler bei 20 rpm für 12 h bei Raumtemperatur inkubiert. Anschließend wurden 0,42µl Salzsäure 37%, reinst (Carl Roth GmbH & Co. KG) hinzufügt. Die Petrischale wurde danach in einen gläsernen Exsikkator überführt und dieser zügig (<10min) auf <300mbar evakuiert. Im verschlossenen Exsikkator wurde dann auf einem Kreisschüttler bei 20 rpm für 6 h bei Raumtemperatur inkubiert. Danach wurde die Flüssigkeit durch Absaugen verworfen und 6,25 ml Aceton zur Membran gegeben. Im wieder verschlossenen Exsikkator wurde danach auf einem Kreisschüttler bei 40 rpm geschüttelt. Diese Schritte wurden je zweimal wiederholt. Abschließend wurde die Membran bei Raumtemperatur und guter Luftzufuhr für mindestens 1 h getrocknet.

Im zweiten Schritt erfolgte die COOH-Funktionalisierung der Membran. Dazu wurde die getrocknete Membran in einer Petrischale mit 5ml DMF überschichtet und 5 min auf einem Kreisschüttler geschüttelt. Die Flüssigkeit wurde durch Absaugen verworfen und der vorangehende Schritt wurde wiederholt. Die Flüssigkeit wurde durch Absaugen wiederum verworfen.

Danach wurde die Membran mit 5 ml Bernsteinsäureanhydrid-Lösung (400mg in 10ml DMF) überschichtet und 10 Min. mit einem Kreisschüttler geschüttelt. Anschließend wurden 5 µl Pyridin zugegeben und es wurde im verschlossenen Exsikkator mit Trockenmittel auf einem Kreisschüttler bei 20 rpm für 12 h bei Raumtemperatur inkubiert. Die Flüssigkeit wurde durch Absaugen verworfen und die Membran mit 5 ml doppelt destilliertem Wasser überschichtet. Es wurde für 5 Min. auf einem Kreisschüttler geschüttelt. Die beiden vorgehenden Schritte wurden wiederholt.

Im dritten Schritt erfolgte die Kopplung von Antikörpern auf die Membran:
Dazu wurde die feuchte Membran aus Schritt 2 mit 5ml MES Puffer (0,05 M Lösung von 19,5 g 2-(N-Morpholino)-ethansulfonsäure, in 1 l H2O, pH=5,5) überschichtet, gefolgt von 5 Min. Schütteln auf einem Kreisschüttler. Der vorhergehende Schritt wird wiederholt. Die Flüssigkeit wurde durch Absaugen verworfen, die Membran wurde mit 5 ml EDC-Lösung (328,6 mg 1-Ethyl-3-[3-dimethylaminopropyl]carbodiimidehydrochloride in 20,53 ml MES Puffer) überschichtet und 10 Min. auf einem Kreisschüttler geschüttelt. Danach wurden 0,5 ml Sulfo-NHS-Lösung (406,2 mg N-hydroxysulfosuccinimide 1,127 ml MES Puffer) hinzugefügt und nach einer Inkubationszeit von 30 Min. wurde die Flüssigkeit abgesaugt und die Membran mit 5ml MES überschichtet. Dann wurde für 10 Min. auf einem Kreisschüttler geschüttelt. Die vorgenannten zwei Schritte wurden wiederholt. Anschließend wurde die Membran mit 2,5ml Antikörperlösung (100 µg /ml anti-Troponin I Antikörper (19C7, HyTest Turku, Finnland) in MES Puffer) überschichtet, und 120 Min. auf einem Kreisschüttler geschüttelt. Die Flüssigkeit wurde wieder durch Absaugen verworfen und die Membran wurde mit 5ml MES inkl. 0,25 % Ethanolamine-Lösung für 30 Min. überschichtet (geblockt). Die Flüssigkeit wurde anschließend verworfen und es wurde mit 5 ml MES Puffer überschichtet, gefolgt von Schütteln für 10 Min. auf einem Kreisschüttler. Der vorgenannten Schritte wurde wiederholt. Schließlich wurde die Flüssigkeit durch Absaugen verworfen und die Membran mit 5ml Trocknungspuffer (PBS pH 7,2, 1% Bovin Serum Albumin, 5 % Saccharose) überschichtet, gefolgt von Schütteln für 60 Min. auf einem Kreisschüttler. Die Flüssigkeit wurde durch Absaugen verworfen und die Membran wurde bei Raumtemperatur unter Belüftung 1 h getrocknet.

### 2. Herstellung der Farbstoffsuspension

Das Partikelkonjugat wurde durch kovalente Kopplung von Antikörpern an die COOH-modifizierte Partikeloberfläche hergestellt. Im ersten Schritt wurden 10µg fluoreszente Mikropartikel (Estapor F1-Z100, Merck KGaA, Darmstadt, Deutschland) durch Zentrifugation bei 7000rpm vom Lagerpuffer getrennt und durch Ultraschallbehandlung in 800µl MES Puffer (ph6) resuspendiert. Im zweiten Schritt wurden 100µl EDC-Lösung (328,6 mg 1-Ethyl-3-[3-dimethylaminopropyl]carbodiimidehydrochloride in 20,53 ml MES Puffer) hinzugefügt und 10 Min. auf einem Kreisschüttler geschüttelt. Danach wurden 100µl Sulfo-NHS-Lösung (406,2 mg N-hydroxysulfosuccinimide 1,127 ml MES Puffer) hinzugefügt und nach einer Inkubationszeit von 30 Min wiederum durch Zentrifugation der Überstannd von den Partikel abgetrennt. Im dritten Schritt wurden die Partikel durch zweimaliges Resuspendieren in 1ml MES und nachfolgendes Zentrifugieren bei 7000rpm gewaschen. Im vierten Schritt wurden die aktivierten Partikel durch Ultraschalleinwirkung in 1ml einer Lösung von 1µg/ml anti-Troponin-I-Antikörper (16A11, Hy Test Ltd Turku Finnland) in MES-Puffer pH=8,0 suspendiert und 1.5h bei RT unter Schütteln inkubiert. Anschliessend wurden 3.5µl einer 0,25 % Ethanolamin-Lösung hinzugefügt und weitere 15min vorsichtig geschüttelt. Abschliessend wurde das Partikelkonjugat durch dreimaliges Zentrifgieren und nachfolgendes Resuspendieren in Block-Puffer (30 mM Phosphat, pH=7,4, 1% Bovines Serum Albumin, 2mM Eisen (II) sulphat, 2 mM EDTA) gereinigt und ein Stammdispersion von 10 µg Partikel pro 1ml PBS Puffer hergestellt.

### 3.Herstellung der Filteranordnung und Pipetierschema

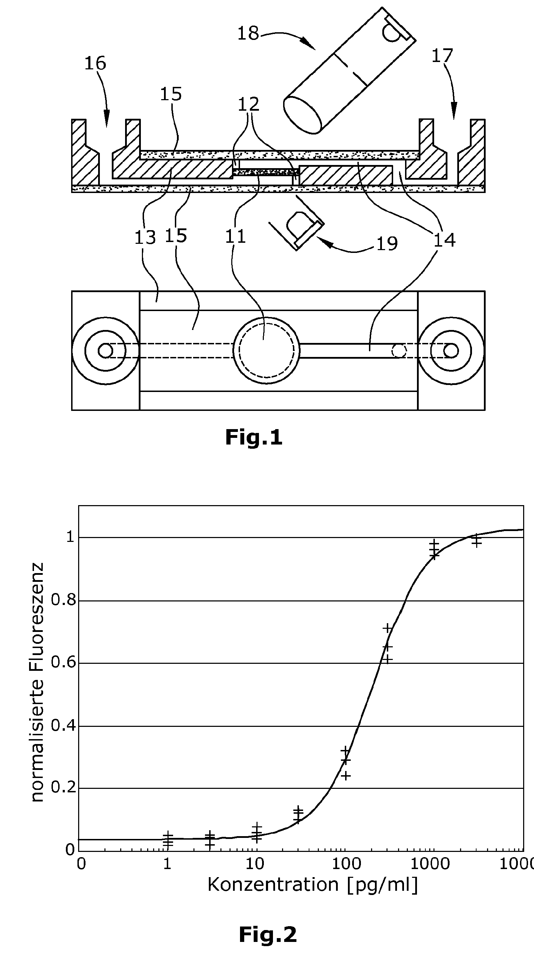

Fig. 1 zeigt einen Querschnitt und eine Aufsicht auf die verwendete Filteranordnung. Die Filteranordnung und Auswerteoptik weist die folgenden Elemente auf: Filtermembran 11, Klemmringe 12, Korpus der Durchflusszelle 13, Flüssigkeitskanäle 14, Transparente Deckel 15, Mini-Luer Zulauf 16, Mini-Luer Ablauf 17, Lichtquelle 18, Photodetektor 19.

Aus der Membran wurden mit Hilfe einer Stanze kreisförmige Filter 11 von 5 mm Durchmesser ausgestanzt. Diese wurden mit Hilfe von Klemmringen 12 in der Kavität einer Durchflusszelle 13 (ChipShop GmbH, Jena) fixiert. Die Ober- und Unterseiten der Kavität sowie der mit ihr verbundenen Flüssigkeitskanäle 14, wurden durch laminieren mit einem transparenten Deckel 15 verschlossen. Die Flüssigkeitskanäle stellen eine Verbindung zu den Mini-Luer-Verbindern des Zulaufs 16 und Ablaufs 17 her. Der Zulauf wurde mittels eines Abschnittes Tygon-Schlauch (SC0047, ISMATEC Laboratoriumstechnik GmbH, Wertheim-Mondfeld, Deutschland) mit einer 750 µl Minisäule (ABICAP, Senova GmbH, Weimar) verbunden. Die Probenzugabe erfolgte direkt in die Minisäule. Es wurden jeweils 500 µl Kontrolllösung humanes Troponin I (8T53, Hytest, Ltd., Turku, Finnland) in PBS Puffer mit den Konzentrationen 0pg/ml, 1pg/ml, 3 pg/ml, 10 pg/ml, 30 pg/ml, 100 pg/ml, 300 pg/ml, 1 ng/ml, 3 ng/ml in die Minisäule pipettiert. Die Durchflusszeit betrug jeweils 6 Min. Anschließend wurden 500 µl der in PBS Puffer (pH=7,4) auf eine Partikelkonzentration von 2µg/ml verdünnten Farbstoffsuspension pipetiert. Nach einem Waschschritt mit 750 µl Waschpuffer (PBS, pH=7,4 0,05 % Tween 20) erfolgte die fluorimetrische Auswertung wie nachfolgend beschrieben.

### 4. Auswerteoptik und Auswertung

Oberhalb der Durchflusszelle ist eine LED-Lichtquelle 18 (Fig. 1) angebracht, die die Membran unter einem Winkel von 45 Grad mit kollimiertem Licht bestrahlt. Die Lichtquelle ist eine 420nm InGaN LED (VL410-5-15, Roithner Lasertechnik, Wien) sowie einer Sammellinse (LB1092, Thorlabs GmbH, Dachau) in einem Tubus (SM05L10, Thorlabs GmbH, Dachau). Unterhalb der Durchflusszelle befindet sich ein Detektor 19 z. B. bestehend aus einer wellenlängenselektiven Photodiode (S6430-01, Hamamatsu Photonics, Herrsching, Deutschland). Der Photostrom wird von einen Transimpedanzverstärker (DLPCA-200, FEMTO Messtechnik GmbH, Berlin, Deutschland) verstärkt und an eine Datenerfassungskarte (PCI-6024E, Agilent Technologies Deutschland GmbH, Böblingen) geleitet.

Die Durchflusszellen wurden in der Auswerteeinheit jeweils fixiert und die Fluoreszenz bestimmt. Figur 2 zeigt die erhaltenen Ergebnisse.

Die doppelte Standardabreichung der normalisierten Fluoreszenz der Nullprobe lag bei 0.03 Damit ergab sich eine rechnerische Nachweisgrenze von 25pg/ml.

### Beispiel 2

### Fluorimetrischer Nachweis von Troponin I im lateral flow Verfahren

### 1. Erzeugung einer mit anti Troponin I funktionalisierten Bindungslinie auf einer PTFE Membran

Im ersten Schritt wurden mit APTS entsprechend Beispiel 1 Aminogruppen auf eine hydrophilisierte PTFE-Membran aufgebracht. Im zweiten Schritt wurde daran in Linienform der anti- Troponin I Antikörper mittels EDC/ NHS Aktivierung gekoppelt. Dazu wurden im Eisbad 1 mg affinitätsgereinigter monoclonaler Antikörper (19C7, HyTest Turku, Finnland) in 1ml MESPuffer (pH=5.2) mit 100 µl einer 4mg/ml EDC Lösung versetzt, 1 Min. geschüttelt und anschließend mit 100 µl einer 11 mg/ml sulfo-NHS-Lösung versetzt und weitere 3 Min. geschüttelt. Der aktivierte IgG Komplex wurde umgehend in den eisgekühlten Reagenzienbehälter des Dispensingsystems (Biodot Ltd., Chichester, UK). Überführt mit dem die Bindungslinie die Membran aufgetragen wurde. Dazu wurde die im ersten Schritt hergestellte Membran auf den Tisch des Gerätes aufgelegt und die im zweiten Schritt erzeigte Antikörperlösung mit einer Beschichtungsrate von 1µl/cm aufgetragen. Die Membran wurde anschließend für 30min in einer Feuchtekammer inkubiert, dann in Trocknungspuffer (PBS mit 1%BSA und 5% Saccharose) überführt und im Exsikkator für 10min entgast. Abschließend wurde die Membran bei 37°C 30 Min. im Trockenschrank getrocknet.

### 2.Herstellung der Filteranordnung und Pipetierschema

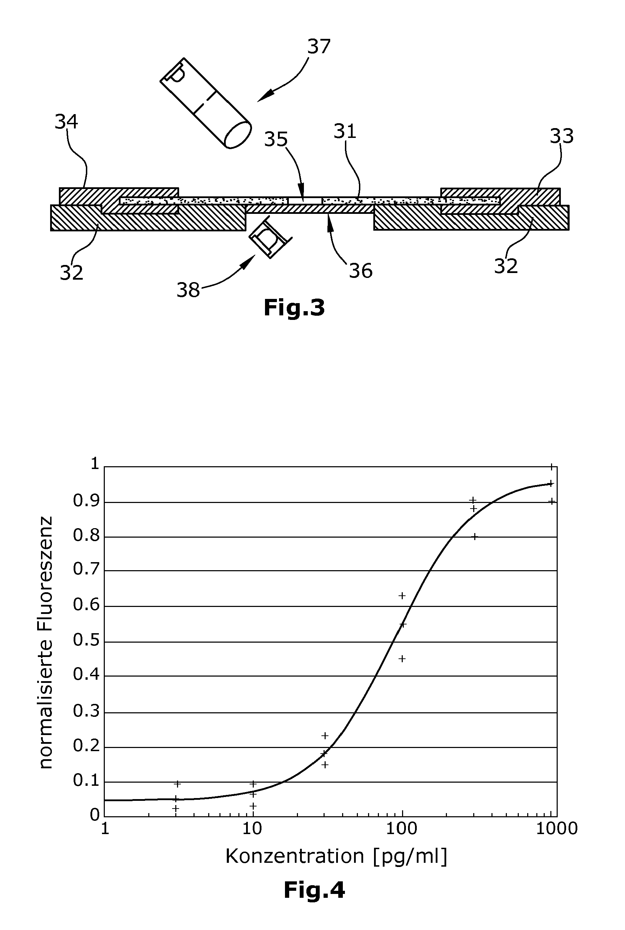

Die Filteranordnung ist in Fig. 3 schematisch dargestellt. Diese Filteranordnung und Auswerteoptik weist die folgenden Elemente auf: Membran 31, Membranhalterung 32, Glasfaserviles 33, Saugpad 34, Bindungslinie (Immobilisierter Antikörper) 35, Deckglas 36, Lichtquelle 37, Photodetektor 38. Die Membran 31 wurde als 5cm langer und 5mm breiter Streifen zugeschnitten und durch Klemmung in einer Lateral-Flow Testkassette 22 (Dima-Diagnostics, Göttingen, Deutschland) fixiert. In den Boden der Testkassette wurde im Bereich der Bindungslinie 35 ein 5x5mm großer Durchbruch erzeugt und dieser durch Einkleben eines handelsüblichen Deckglases 26 verschlossen. An den Enden der Membran sind ein Glasfaservlies 33 (Dima-Diagnostics, Göttingen, Deutschland) und ein saugfähiges Cellulosepad 34 (Whatman, USA) angebracht. Im oberen Teil der Testkassette befindet sich eine Vertiefung zur Aufgabe von Probenmaterial auf die Membran.

Es wurden in einem Eppendorfröhrchen jeweils 250 µl Kontrolllösung humanes Troponin I (8T53, Hytest Ltd., Turku, Finnland) in PBS Puffer pH= 7,4 mit den Konzentrationen 0pg/ml, 1pg/ml , 3 pg/ml, 10 pg/ml, 30 pg/ml, 100 pg/ml, 300 pg/ml, 1 ng/ml, mit 50 µl der in PBS-Puffer auf 5µg Partikel pro ml verdünnten Farbstoffsuspension aus Beispiel 1 gemischt und 5 Min. inkubiert. Nachfolgend wurden davon 250 µl in die Vertiefung der Testkassette pipettiert. Nach 10 Min. wurden 100 µl Waschpuffer pipettiert.

### 3.Auswerteoptik und Auswertung

Zur optischen Auswertung wurde die Testkassette in die Auslesevorrichtung aus Beispiel 1 gespannt und die Fluoreszenz bestimmt. Figur 4 zeigt eine typische Kalibrationskurve. Bei dem Verfahren wird die Nachweisgrenze bei ca. 80 pg/ml erreicht.

### Beispiele 3

### Durchflussassay zum Nachweis von Troponin

### 1. Herstellung des Trägers und Immobilisierung der Antikörper

An ein poröser PMMA Sinterformkörper (2,5mm Höhe x 5 mm Durchmesser) mit einem Porenanteil von 40% und einer mittleren Porengröße von 10µm (Durst Filtertechnik, Biesigheim, Deutschland), wurden in Anlehnung an die in [Meyer M.F, et al, Biosensors and Bioelectronics 2007,22(6)p.973] beschriebene Methode Antikörper durch Physisorption immobilisiert

Dazu wurden 50 der Formkörper in ein 50 ml Becherglas mit 20 ml Ethanol (96%) gegeben und unter leichtem Rühren auf einem Magnetrührer 20 min bei 10 Torr in einem Exsikkator entgast. Anschließend wurden die Filter 3-mal mit 50%iger Ethanollösung gewaschen und abschließend in 10 ml Karbonatpuffer (100 mM, pH= 9,5) entgast. Anschließend wurden 500 µl einer 1mg/ml anti-Troponin (19C7, HyTest Turku, Finnland), Lösung in Karbonatpuffer unter Rühren zugegeben. Die Filter wurden im Exsikkator 6h bei Zimmertemperatur unter leichtem Rühren inkubiert. Anschließend wurde die Antikörperlösung durch eine Blocklösung 5,5 mg/ml Casein in PBS Puffer (150 mM, pH7,3) ersetzt und nach Entgasen 1 h unter rühren geblockt. Die Membranen wurden bei Raumtemperatur in einem Wirbelbetttrockner (FPD200 Endicotts, London, UK) 10 min getrocknet.

### 2. Filteranordnung und Pipettierschema

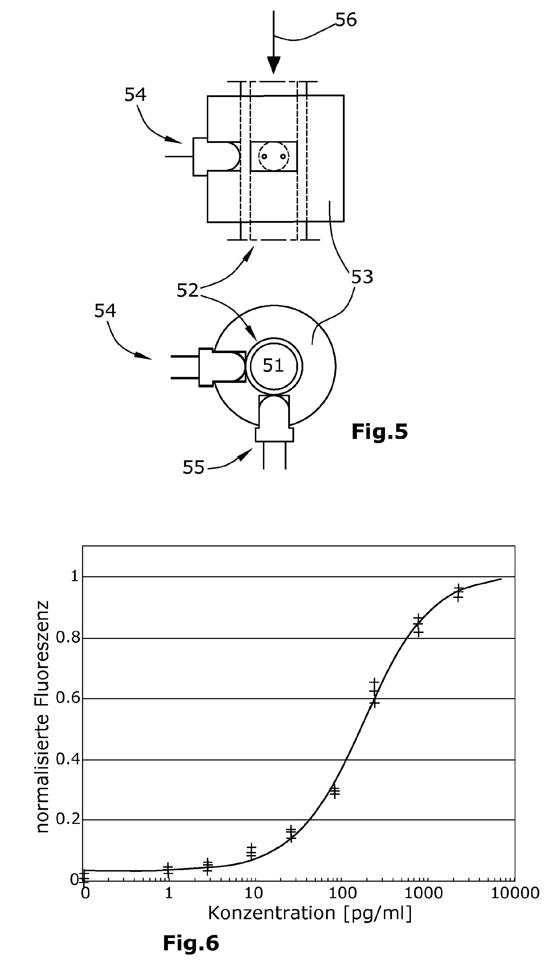

Figur 5 zeigt einen radialen und einen axialen Schnitt durch die verwendete Anordnung. Diese Durchflussordnung für Miniatursäulen weist die folgenden Elemente auf: Filter 51, Minitur-Säule 52, Messkopf 53, LED 54, Photodiode 55, Strömungsrichtung 56. Die beschichteten Sinterformkörper 51 wurden in jeweils eine Minisäule-Säule 52 (ABICAP, Senova GmbH, Weimar) eingesetzt. In diese wurden jeweils 500 µl Kontrolllösung humanes Troponin I (8T53, Hytest, Ltd., Turku, Finnland) in PBS Puffer mit den Konzentrationen 0pg/ml, 1pg/ml, 3 pg/ml, 10 pg/ml, 30 pg/ml, 100 pg/ml, 300 pg/ml, 1 ng/ml, 3 ng/ml in pipettiert. Nach Durchfluss der Probe durch die Anordnung wurden 500µl der in PBS Puffer auf 2µg/ml verdünnten Farbstoffsuspension und anschließend 3x 250µl Waschpuffer gegeben. Anschließend wurden zur Anpassung der Brechzahlen 500µl einer Mischung von 83% Thiodiglycol in Wasser in die Säule pipetiert. Die Fluoreszenzmessung wurde ausgelöst, nachdem die Mischung die Poren des Sinterfomkörpers vollständig infiltriert hatte.

### 3. Auswerteoptik und Auswertung

Zur Messung wurde die Säule 52 in einen modifizierten Messkopf eines ABICAP-Readers (Senova GmbH, Weimar, Deutschland) eingebracht. Der Messkopf 53 besteht aus einem schwarz eingefärbten Kunststoffzylinder, mit einer axialen Bohrung, in der sich die Säule befindet, und zwei radialen Bohrungen unter einem Winkel von 90 Grad in der Ebene der Sinterformkörpers 51 In den radialen Bohrungen befinden sich eine 420nm InGaN LED 54 (LED420-33, Roithner Lasertechnik GmbH, Wien) sowie eine wellenlängenselektive Photodiode 55 (EPD-660-3-0.5, Epigap Optoelectronics, Berlin, Deutschland). Der Photostrom wird von einen Transimpedanzverstärker (DLPCA-200, FEMTO Messtechnik GmbH, Berlin, Deutschland) verstärkt und an eine Datenerfassungskarte (PCI-6024E, Agilent Technologies Deutschland GmbH, Böblingen) geleitet

Die Figur 6 zeigt die erhaltenen Kalibrationskurve dieses Assays.

Die doppelte Standardabreichung der normalisierten Fluoreszenz der Nullprobe lag bei 0.02 Damit ergab sich eine Nachweisgrenze von 8 pg/ml.

## Patentansprüche

1. Poröser Träger für heterogene Bindungsassays, wobei der Träger immobilisierte Bindungsmoleküle zum Binden von Analyten oder Konjugaten sowie Poren aufweist, die ein Porenvolumen besitzen und die Poren in einer festen Phase angeordnet sind, mit der Maßgabe, dass die feste Phase einen Brechungsindex aufweist und das Porenvolumen einen Brechungsindex aufweist, **dadurch gekennzeichnet, dass** eine Differenz des Brechungsindex der festen Phase und des Brechungsindex des Porenvolumens < 0,05, insbesondere <0,02 ist.

2. Träger nach Anspruch 1, **dadurch gekennzeichnet, dass** der Träger aus einem porösen Polymermaterial besteht.

3. Träger nach Anspruch 2, **dadurch gekennzeichnet, dass** das poröse Trägermaterial aus einem porösen Polymermaterial besteht, das ausgewählt ist aus der Gruppe bestehend aus fluoriertem oder perfluoriertem Polymeren, nanoporösem Siliziumdioxid, Polyethylen, Cellulose, Nitrozellulose, Polyamid, Polymethacrylat, mineralische Gläsern bestehen

4. Träger nach Anspruch 3, **dadurch gekennzeichnet, dass** das fluorierte oder perfluorierte Polymere perfluoriertes Ethlen-Propylen-Copolymer (FEP), Polytetrafluoroethylen (PTFE), Hexafluoropropylenvinylidenefluorid (THV), Perfluoroalkoxyalkanen (PFA), sulfoniertem Polytetrafluorethylen (NAFION) ist.

5. Träger nach mindestens einem der Ansprüche 1 bis 4, wobei der poröse Träger als Membran oder Formkörper ausgestaltet ist.

6. Träger nach Anspruch 5, wobei der als Formkörper ausgestaltete poröse Träger eine Fritte ist.
